# EUROPEAN PATENT APPLICATION

(11) **EP 2 036 585 A1**
(43) Date of publication of application: **18.03.2009**
(21) Application number: 07116299.4
(22) Date of filing: 13.09.2007
(51) Int. Cl.: A61M 5/142, F04B 43/12, A61M 5/00

(54) **Roller pump**

(71) Applicant: Ulrich GmbH & Co. KG, 89081 Ulm (DE)
(72) Inventor: Baecke, Dr. Martin, 06847 Dessau (DE); Zupp, Andre, 06844 Dessau (DE); Schwerdtfeger, Uwe, 39439 Amesdorf (DE)
(74) Representative: Hellmich, Wolfgang

(57) **Abstract**

The invention relates to a roller pump which comprises an abutment (31, 52), at least one roller (51) and a casing (2, 3, 6). A pump hose (42) is squeezed between said roller (51) and said abutment (31, 52). A hinge (5) connects said abutment (31, 52) and said casing (2, 3, 6) pivotably, the axis of said hinge (5) being parallel to the plane of said pump hose (42). The invention further relates to a roller pump which comprises a resilient roll member (52) which is fixed to said abutment (31, 52). The pump hose (42) is pressed against said resilient roll member (52). The invention additionally relates to a roller pump which comprises two roller gears (37; 162). Each roller gear being torque-proof connected to one of said two rollers (51; 169). The two roller gears (37; 162) engage with another gear (33; 161, 160) for ensuring zero relative velocity of the portion of said rollers (35; 142) squeezing said pump hose (42) with respect to the squeezed portions of said pump hose (42). The invention finally relates to a roller pump which comprises a drive train (71, 72, 73) for mechanically connecting a motor (21) and said rollers (51; 169). The drive train (71, 72, 73) comprises a sliding hub (73) for limiting the transmitted torque.

## Description

The present invention pertains to peristaltic pumps and more specifically to roller pumps for medical purposes.

In particular in medical technology peristaltic and roller pumps are popular, since the aseptic part of the pump, which is the pump hose, is replaced from time to time without the need to sterilize any part of the pump including the pump hose. The pump hose may actually be a part of the pouch which comprises the liquid to be delivered to a subject.

The US 4,867,744 discloses a peristaltic linear pump, which is particularly useful as a blood pump. The pump is equipped with a series of rollers eccentrically mounted upon a drive shaft. The pump also includes an elastomeric fluid delivery tube, which is supported to extend in a direction parallel with the axis of the drive shaft. Each roller has a generally cylindrical outer surface equipped with a concentric annular rim that projects radially outwardly beyond the cylindrical surface to occlude a section of the tube when that roller is at its perigee with respect to the tube.

The DE 198 08 590 discloses a humidifying system. A hose or peristaltic pump delivers water from a commercial water pouch through an evaporator to a breathing air conduit.

The US 5,062,775 describes a roller pump in an extracorporeal support system also for pumping blood through a blood treatment device such as an oxygenator. The roller pump has a housing with a keyhole-shaped recess, which has a throat and an arcuate recessed surface. The opposite sides of the throat connect with opposite ends of the arcuate recessed surface. An axle is centrally positioned within the recess with respect to the arcuate recessed surface. A pump head is removably secured to the axle and positioned within the recess. The pump head comprises a base and an upper member for holding two rollers. The rollers are each formed to be conical in shape with an outer surface positioned to be proximate the arcuate recessed surface. A tube is positioned between the outer surface of the roller and the arcuate recessed surface to be pressed or urged against the arcuate recessed surface by the roller. Upon rotation, the roller rolls over the tube squeezing it and thereby urging liquid or fluid in the tube therethrough. Since both, the rollers and the arcuate recessed surface are conical, the distance between the outer surfaces of the rollers and the arcuate recessed surface may be adjusted by moving the pump head axially toward and away from the axle and the arcuate recessed surface. The angle between the central axle and the arcuate recessed surface is approximately 30°. The angle between the surface of a roller and the axis of a roller is approximately 17°. For the axial movement of the pump head a screw, which may be operated by a screwdriver, and a spring are provided. A key fixes the pump had to the central axle. The key may be shifted between a locked and an unlocked position. In the unlocked position, the pump head may be removed for example for replacing the tube.

Roller pumps are also used for administering contrast agents to subjects in order to provide superior image quality when carrying out CT and MRI scans. Injectors ulrich ohio tandem, ulrich missouri, ulrich ohio M, ulrich mississippi and Swiss Medical Care CT Exprés come with roller pumps which pump contrast agent and/or saline solution from one of at least two different containers into the subject. This architecture enables the application of several injections consecutively from one contrast agent container.

CT, originally known as computed axial tomography (CAT or CT scan) and body section roentgenography, is a medical imaging method for generating a three-dimensional image of the internals of an object from a large series of two-dimensional X-ray images taken around a single axis of rotation. The word "tomography" is derived from the Greek tomos (slice) and graphein (to write). CT produces a volume of data which can be manipulated, through a process known as windowing, in order to demonstrate various structures based on their ability to block the X-ray beam. Modern CT scanners allow this volume of data to be reformatted in various planes or even as volumetric (3D) representations of structures.

Magnetic resonance imaging (MRI), formerly referred to as magnetic resonance tomography (MRT) is a non-invasive method used to render images of the inside of an object. It is primarily used in medical imaging to demonstrate pathological or other physiological alterations of living tissues.

From an injector point of view, there is no big difference between CT and MRI. However, injectors for MRI must not be disturbed by high magnetic fields in the range of 1 T and the injectors must not disturb the magnetic field of the MRI. For the latter reason, injectors for MRI are often powered by batteries or direct current (DC).

In the following we will focus on the injectors ulrich missouri and ulrich mississippi, which are identical apart from the power supply. Ulrich mississippi is battery-powered which enables MRI application.

A core element of the ulrich injectors is a cross-shaped hose system for delivering contrast agent and saline solution from one of a left container for a first contrast agent, a middle container for saline solution and a right container for a second contrast agent to the subject.

The cross-shaped hose system comprises a left contrast agent hose, a saline solution hose, a right contrast agent hose and a pump hose which are connected by a cross connector. The pump hose is fixed by a mounting and guidances to the casing of the injector. The hose system is replaced after each working day or after 24 hours of operation.

The roll pump consists of a pump wheel and two wings. The pump wheel comprises three rolls and three guidances, which have the form of circular arcs. Each of the wings comprises a circular-arc-shaped abutment. The axes of the circular-arc-shaped abutments coincide with the central axis of the pump wheel. The rolls have a cylindrical shape. During operation the pump wheel turns clockwise, whereas the rolls turn counterclockwise. At least one of the rolls squeezes the pump hose against one of the abutments so that no fluid may flow through the squeezed portion of the pump hose. When the pump wheel turns clockwise, the squeezed portion moves towards the subject thereby pumping fluid towards the subject.

For inserting and removing the hose system, the wings may be swung outward and downward by about 45° around a pivot. For cleaning purposes the wings may be detached. The rolls are adjusted to the wings and in particular the circular-arc-shaped abutments. Therefore, the wings of different injectors must not be exchanged for example during cleaning by the operating personal.

It is the object of this invention to provide a roller pump, which is serviced more easily.

This object is achieved by the subject matter of the independent claims.

Preferred embodiments of the invention are the subject matter(s) of the dependent claims.

In the following preferred embodiments of this invention are described referring to the accompanying drawings. In the drawings:
Fig. 1 shows a top view of an injector head which comprises a first embodiment of an inventive roller pump;
Fig. 2 shows a bottom view of the injector head shown in figure 1;
Fig. 3 shows a cross-section along the line A-A in figure 2;
Fig. 4 shows a perspective bottom view of the partly disassembled injector head shown in figure 1;
Fig. 5 shows a perspective bottom view of the partly disassembled injector head shown in figure 1;
Fig. 6 shows a front view of a second embodiment of the drive train of the roller pump shown in figures 3 to 5;
Fig. 7 shows a second embodiment of an inventive roller pump for an injector head;
Fig. 8 shows a closing gear stage in an open state;
Fig. 9 shows a cap in an open state;
Fig. 10 shows the cap with rollers in an open state;
Fig. 11 shows a closing gear stage in a closed state;
Fig. 12 shows a cap in a closed state;
Fig. 13 shows the cap with rollers in a closed state;
Fig. 14 shows a third embodiment of an inventive roller pump for injector head;
Fig. 15 shows a detailed view of the planetary drive of the third embodiment; and
Fig. 16 shows a detailed view of the movable cap and the sliding blocks of a third embodiment.

While the present invention is described with reference to the embodiments as illustrated in the following detailed description as well as in the drawings, it should be understood that the following detailed description as well as the drawings are not intended to limit the present invention to the particular illustrative embodiments disclosed, but rather the described illustrative embodiments merely exemplify the various aspects of the present invention, the scope of which is defined by the appended claims.

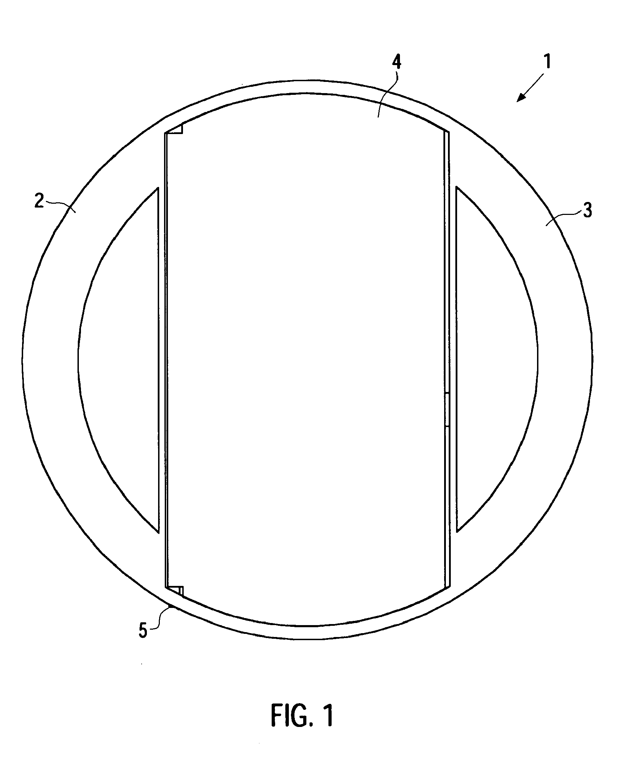

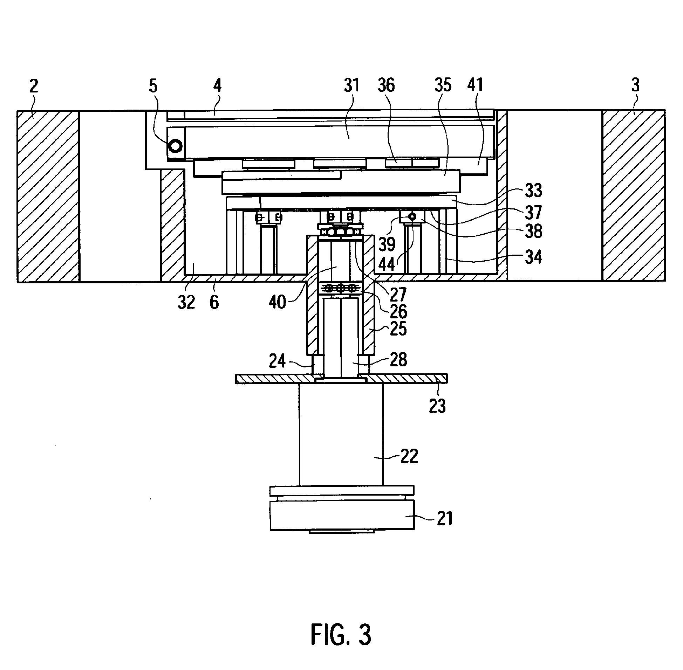

Figure 1 shows a top view of an injector head 1, which comprises a first embodiment of an inventive roller pump. The injector head 1 is a shaped part and may be made of polyurethane. The injector head 1 comprises two handles 2 and 3 on the left and right-hand sides. A design cap 4 covers the inventive roller pump in figure 1. A hinge splint pin 5 pivotally attaches an abutment 31 to the injector head 1. The abutment 31 is shown in more detail in figures 3 to 5 and may be made of stainless steel.

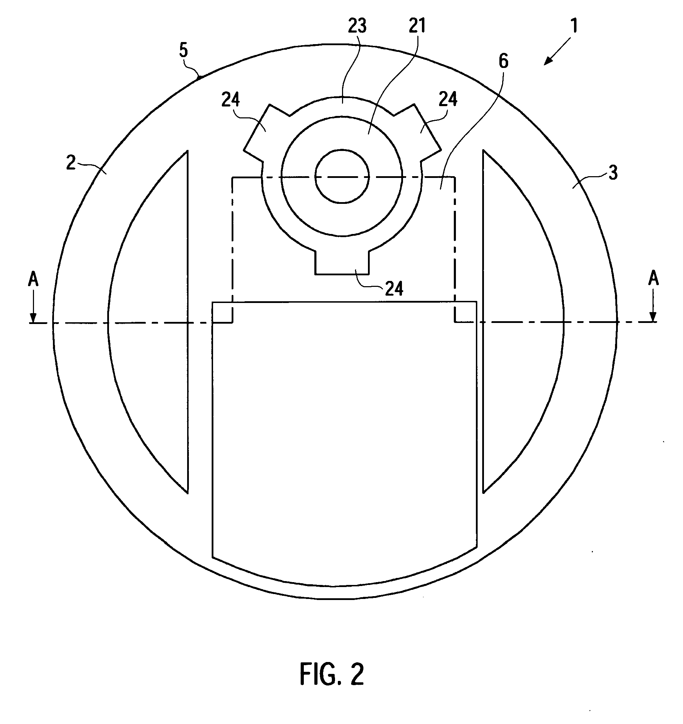

Figure 2 shows a bottom view of the injector head 1 including the two handles 2 and 3, and a corner of the hinge splint pin 5. In addition, figure 2 shows a motor 21, a discoidal gearbox support 23 and three holders 24. The bottom of the injector head below the roller pump is designated with reference numeral 6.

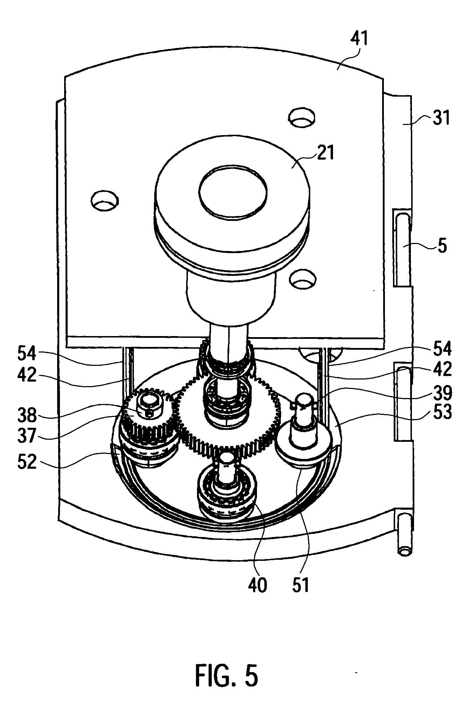

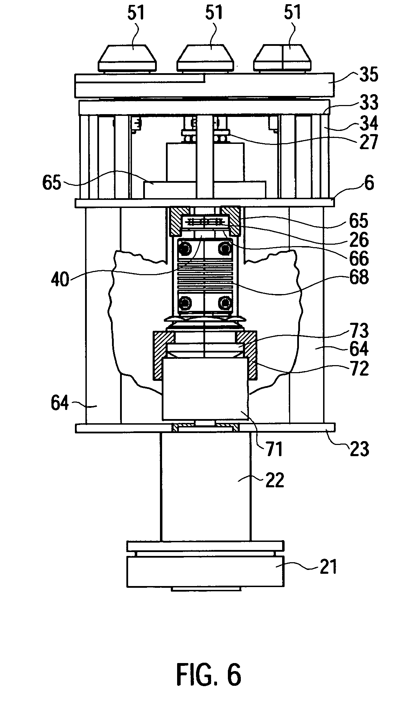

Figure 3 shows a cross-section through the injector head 1 along the line A-A shown in figure 2. In figure 3 the pump itself is hidden within the abutment 31, but will be shown in figures 5 and 6. Only the cylindrical portions of the rollers 36 are visible in figure 3 in front of the disposable 41. The four rollers 36 (one is hidden) are turnably mounted within roller carrier 35. An internal toothing gear 33 together with satellites 37 insurers zero relative velocity between the rollers and a pump hose. Below the internal toothing gear 33 fastening portions 38 of the satellites 37 and lower ends of roller shafts 44 of the rollers 36 are visible. Shear splint pins 39 provide a torque prove connection between the satellites 37 and the shafts 44.

The internal toothing gear 33 is fixed to the bottom 6 of the injector head 1 by eight columns 34. Four of the columns 34 are visible in figure 3. The injector head 1 comprises a sleeve portion 25 for fixing a ball bearing 26 and an axial ball bearing 27. The axial ball bearing 27 accepts the vertical downward force from the rollers 36. The ball bearing 26 accepts radial forces and insurers axial alignment of a central shaft 40.

Only one of the three holders 24 is visible in figure 3. The three holders 24 mechanically connect the gearbox support 23 to the bottom 6. A gearbox 22 abuts or is mounted to the gearbox support 23. The motor 21 is mounted to the gearbox 22. A coupler 28 provides a torque-proof connection between a drive axle (not shown) of the gearbox 22 and the central shaft 40.

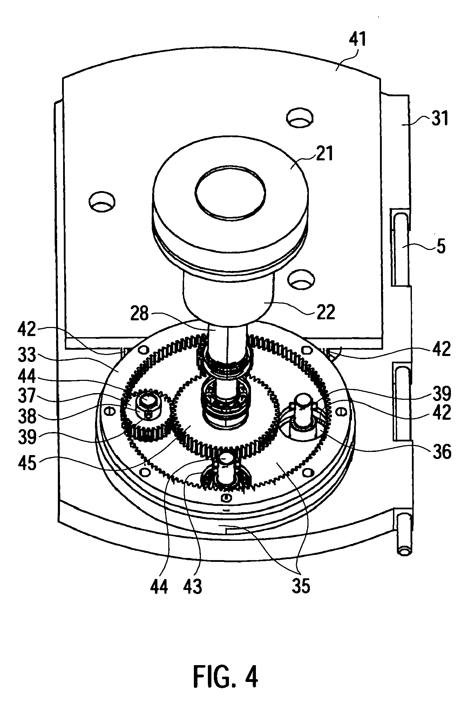

Figure 4 shows a perspective bottom view of the partly disassembled injector head 1. The casing of the injector head 1 including the handles 2 and 3, the bottom 6, the gearbox support 23, the three holders 24, the sleeve portion 25 and the columns 34 are not shown in figure 4. In figure 4 a sun gear 45 becomes clearly visible. The sun gear 45 is driven by the motor 21, the gearbox 22, the coupler 28 and the central shaft 40.

Furthermore, the roller assemblies are disassembled to different degrees in order to show the construction of the roller assemblies. The roller assembly on the left-hand side is complete. Therefore, the satellite 37 with its fastening portion 38, a roller shaft 44 and a shear splint pin 39 are visible. The satellite has been taking off from the front roller assembly. Consequently, a ball bearing 43 for turnably securing the respective roller shaft 44 to the roller carrier 35 becomes visible. The ball bearing has been additionally removed from the roller assembly on the right-hand side. Consequently, the bottom side of a roller 36 and a small part of a pump hose 42 are visible.

In figure 5 the internal toothing gear 33 and the roller carrier 35 have been additionally taking off. Consequently, the conical portions of the rollers 51 are visible. Moreover the biggest part of the pump hose 42 is visible. Further, a conical recess 53 and guidances 54 within the abutment 31 are uncovered. The guidances 54 guide the pump hose 42 from the disposable 41 to the conical recess 53 and back to the disposable 41. The lower part of the conical surface of the conical recess 53 is covered by a resilient rubber roll member 52 whose purpose is to compensate tolerances.

The conical portions 51 of the rollers press the pump hose 42 against the rubber roll member 52. Thereby, the pump hose 42 is squeezed off at at least one position. If the rollers are moved clockwise or counterclockwise, the one or more squeezed-off portions of the pump hose 42 move, which delivers liquid within the pump hose 42. Thicker parts of the pump hose 42 are pressed deeper into the rubber roll member 52. The rubber roll member 52 may also be designed to compensate tolerances within the injector head 1, the abutment 31, the conical portions 51 of the rollers etc.

Since the abutment 31 is hinged by hinge splint pin 5, the abutment 31 may easily be opened. Then the disposable 41 together with the pump hose 42 may be easily replaced. On the opposite side of the hinge splint pin 5 a lock is provided which is not shown in the figures. The lock mechanically connects the abutment of 31 with the injector head 1.

Figure 6 shows a front view of a second embodiment of the drive train of the roller pump shown in figures 3 to 5. The main difference is that the second embodiment comprises a sliding hub 73 for a limiting the torque. As a consequence, the drive train becomes longer. Therefore, the holders 64 in figure 6 are longer than the holders 24 in figure 3. Again, the holders 64 mechanically connect the gearbox support 23 with the bottom 6. The gearbox 22 is mounted to the gearbox support 23. A motor collar 71 is torque-proof connected to an axle of the gearbox 22. A collar 72 presses a sliding hub 73 against the motor collar 71. This connection between the sliding hub 73 and the motor collar 71 limits the torque. A compensating coupler 68 transmits the torque from the sliding hub 73 to the central shaft 40.

The ball bearing 26 is retained by a snap ring 66 within a sleeve portion 65. Above the bottom 6 and apart from the modified sleeve portion 65 the embodiment shown in figure 6 is identical to the embodiment shown in figures 3 to 5.

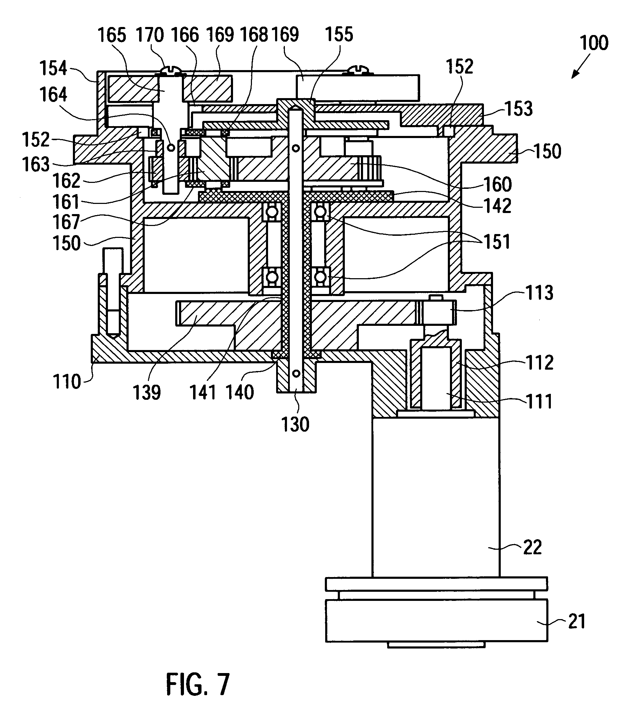

Figure 7 shows a second embodiment of an inventive roller pump 100 for an injector head. The roller pump 100 comprises a lower pump unit 110 and an upper pump unit 150 which are mounted together by screws or stud screws as shown on the left-hand side. The roller pump 100 may be screwed to columns of the injector head or mounted into the injector head by stud screws. Again, the motor 21 is mounted to the gearbox 22 which in turn is mounted to the lower pump unit 110.

A drive axle 111 extends out of the gearbox 22 into a coupling 112. A driver 113 (Mädler GmbH: item number 29301400) is fixed by a bolt to the upper, cylindrical end of the coupling 112. The drive axle 111 and the coupling 112 are torque-proof connected.

A big gear 139 engages with the driver 113. The big gear 139 is torque-proof connected to a sleeve portion 141 of a closing gear support 140. The sleeve portion 141 is pivot-mounted by two grooved ball bearings 151 within the upper pump unit 150. The torque is transmitted to a support portion 142, which constitutes part of kind of a planetary drive. The sun gear 160 is non-pivotably fixed to the lower pump unit 110 by a sun gear shaft 130. There are two kinds of satellites, namely closing gears 161 and roller gears 162. The closing gears 161 are pivotably mounted on the support portion 142. The shaft 165, to which the sleeve portion 163 of the roller gear 162 is non-pivotably fixed by pin 164, are pivotably mounted within a lower arm 167 and an upper arm 168. The lower and upper arms 167 and 168 are also pivotably mounted at the pivot of the respective closing gear 161. Consequently, the shaft 165 may move a short distance in radial direction to the central axis defined by the sun gear shaft 130. The radial movement is limited by a long hole 166 within a movable cap 153.

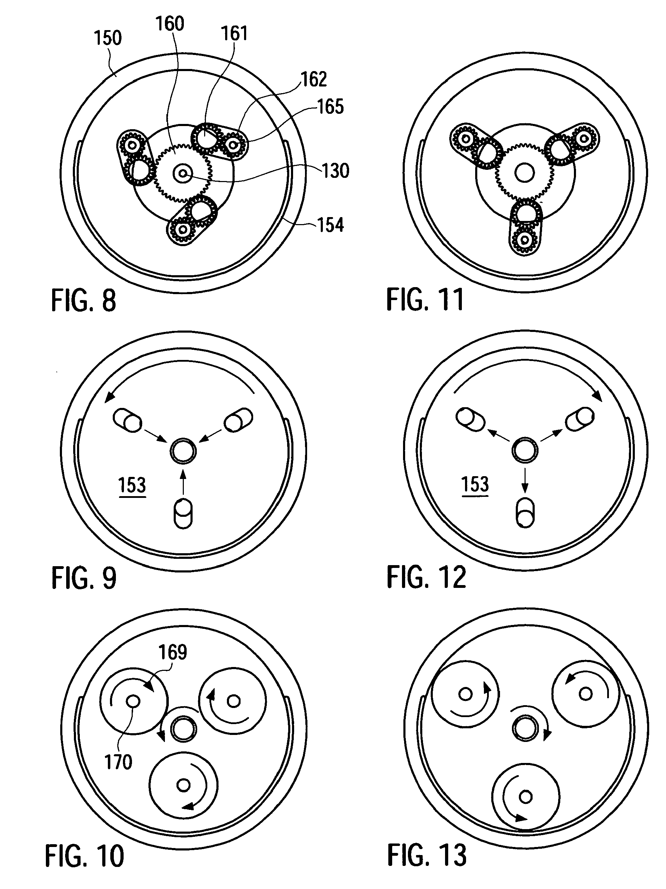

The purpose of this construction is to squeeze a pump hose (not shown) between an abutment 154 and a roller 169, if the support portion 142 turns clockwise and to free the pump hose by moving the rollers 169 to the center defined by sun gear shaft 130, if the support portion 142 turns counterclockwise. This operation will be explained in connection with figures 8 to 13 in more detail.

In figure 7 the movable cap 153 is pivotably mounted by a thin section bearing 152 to the upper pump unit 150. The rollers 169 are non-pivotably mounted by screws 170 to a respective shaft 165. An inner cap 155 is fixed by the shafts of the roller gears 162 to the support portion 142. The bearings of the closing gears 161, arms 167 and 168, and shafts 165 are sleeve bearings in the embodiment shown in figure 7.

In the embodiments described above, the planetary drive could be replaced by a belt drive, which transmits the torque from a central shaft to satellite shafts by one or more belts for each satellite shaft.

In yet another embodiment each roller could be driven by a separate motor. A gear is fixed to each shaft of each motor. Each gear engages with an outer gear having an inner toothing for synchronizing motors and concentrating the mechanical power of all motors on the engaging rollers. The non-engaging rollers basically run free and require a neglectable amount of power.

In yet a further embodiment, the pump hose could run around a cone. Cone-shaped rollers squeeze the pump hose against the cone. The code defines a central axis. The axises of the cone-shaped rollers intersect with the central axis perpendicularly. Bevel gears transmit the mechanical power from a central shaft aligned with the central axis to the cone-shaped rollers.

In the embodiment above the number of rollers could be different from four, more specifically three as in the following embodiments or any other number.

Figures 8 to 13 illustrate the operation of the second embodiment of an inventive roller pump. In figures 8 to 10 the rollers 169 and the respective shafts 165 are drawn to the center. This movement towards the center is limited by the long holes 166 within the movable cap as shown in figure 9. The movement of the support portion 142 and movable cap 153 is or has been counterclockwise, whereas the rollers turn clockwise.

In figures 11 to 13 the rollers 169 and the respective shafts 165 are moved outward. Again, this movement is limited by the long holes 166. However, another limitation may be the slightly varying thickness of the pump hose or other tolerances within the roller pump. Therefore, during normal operation, the outward movement of a shaft 165 does not reach the outer limit defined by the long holes 166 when the corresponding roller 169 presses the pump against the abutment. This is the case, if the roller is in the lower half of the upper unit 150. In figures 11 to 13 the support portion 142 and the movable cap 153 turn clockwise, whereas the rollers turn counterclockwise in order to ensure zero relative velocity between the squeezing rollers 169 and the pump hose.

Also in the second embodiment, which was explained in connection with figures 7 to 13, the side of the abutment 154 next to the pump hose could be covered by a resilient rubber member for compensating tolerances. In other embodiments, the rollers could be made of resilient rubber or covered with resilient rubber. Although the rollers have a cylindrical shape and the abutment is part of a shell of a cylinder in the second embodiment, the rollers and/or the abutment could also have a conical shape in the second embodiment.

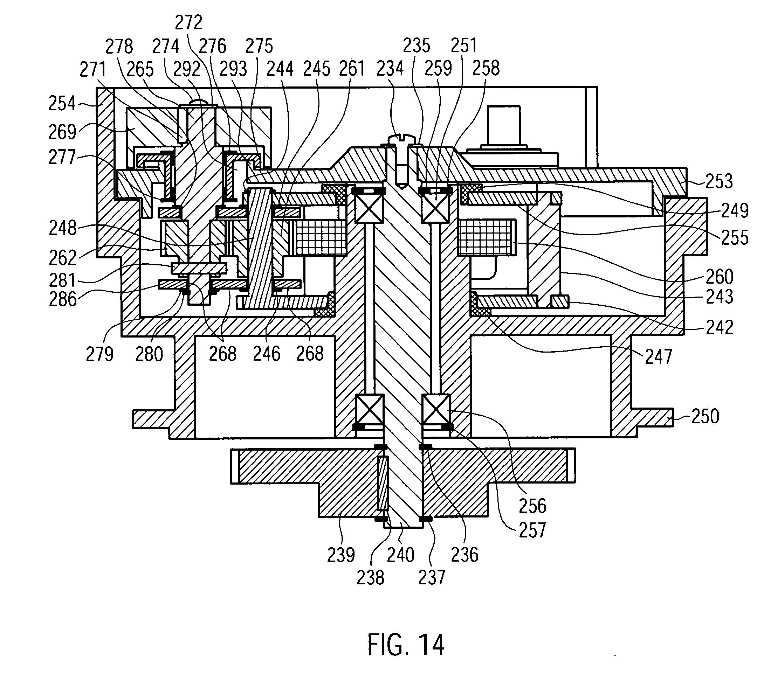

Figures 14 to 16 show a third embodiment of an inventive roller pump. The motor 21 (Maxon EC90 item number 323772), the gearbox 22 (Maxon GP52C item number 223087), the coupling 112, the driver 113 and the lower pump unit 110 are identical to the second embodiment shown in figure 7 and are consequently not shown in figure 14 again. The reference numbers of similar parts of the second and third embodiment differ by 100.

The most significant difference between the second and third embodiment that the sun gear shaft 130 is not necessary in the third embodiment, rather the sun gear 260 is fixed to a sleeve portion of an upper pump unit 250. Roller gears 262 are driven by a movable cap 253 and a drive shaft 240. This constructions further obviates the need for a thin section bearing.

Within the sleeve portion of the upper pump unit 250 the drive shaft 240 is supported by an upper ball bearing 251 (DIN 625-1-6001) and a lower ball bearing 256 (DIN 625-1-6001). The upper ball bearing 251 is fixed to the sleeve portion of the upper pump unit 250 by a locking ring 258 (DIN 472-28x1,2) and to the drive shaft 240 by a locking ring 259 (DIN 471-12x1). The lower ball bearing 256 is fixed to the sleeve portion by a locking ring 257 (DIN 472-28x1,2). DIN is short for Deutsches Institut für Normungen e.V. (German Institute for Standards). Items specified by DIN or item numbers may be reworked e.g. by drilling holes for pins or milling groves for feather keys. So such items are not necessarily identical to the ones shown in the figures.

A big gear 239 (Mädler GmbH: item number 29310000) is torque proof fixed to the drive shaft 240 by a feather key 238 (DIN 6885-A-3x3x16) and two locking rings 236 and 237 (DIN 471-12x1).

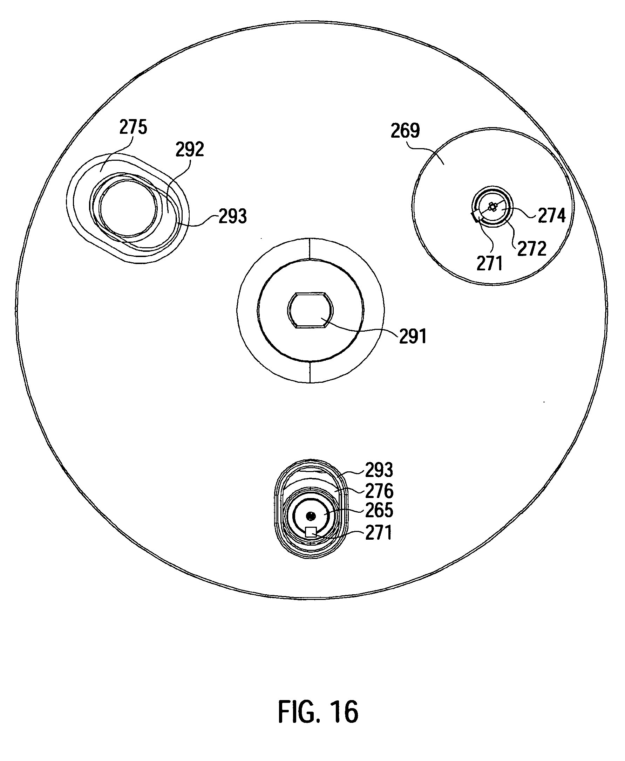

At the upper end of the drive shaft 240, a movable cap 253 is torque proof fixed to this upper end by a washer 235 (DIN 125-A-4,3 HV-A2) and a lens head screw 234 (DIN EN ISO 7045 M4x6). The form fit between the drive shaft 240 and the movable cap 253 is shown in more detail in figure 16.

A support portion 242 is pivot mounted by a slide bearing 247 to the outside of the sleeve portion. The support portion 242 is fixed to an inner cap 255 by three arm shafts 248, three locking rings 244 (DIN 471-6x0,7) and three spacers 243. The arm shafts 248 form an integral part of the support portion 242. The inner cap 255 is supported by a slide bearing 249.

On each of the three arm shafts 248, an arm 268 is pivot mounted by two slide bearings 245 and 246 (Igus GmbH: Iglidur GFM-1011-026). In addition, a closing gear 261, which engages with the fixed sun gear 260 (Mädler GmbH: item number 29304800) and a respective roller gear 262, is pivot mounted on each arm shaft 248.

On the outer ends of each arm 268 a roller shaft 265 is supported by a slide bearing 278 (Igus GmbH: Iglidur GFM-1011-026) and 279 (Igus GmbH: Iglidur GFM-0607-024). The roller shaft 265 is secured by a locking ring 280 (DIN 471-6x0,7) to the slide bearing 279 and a lower portion 286 of a respective arm 268. A roller gear 262 is torque proof fixed by a breaker bolt 281 to a respective roller shaft 265. A sliding block 275 is pivot mounted on each of the roller shafts 265 by slide bearings 276 and 277 (both: Igus GmbH: Iglidur GFM-1416-04). On top of each roller shaft 265 a roller 269 is fixed by a feather key 271 (DIN 6885-A-3x3x10), a washer 272 (DIN125-A-4-3 HV-A2) and a screw 274.

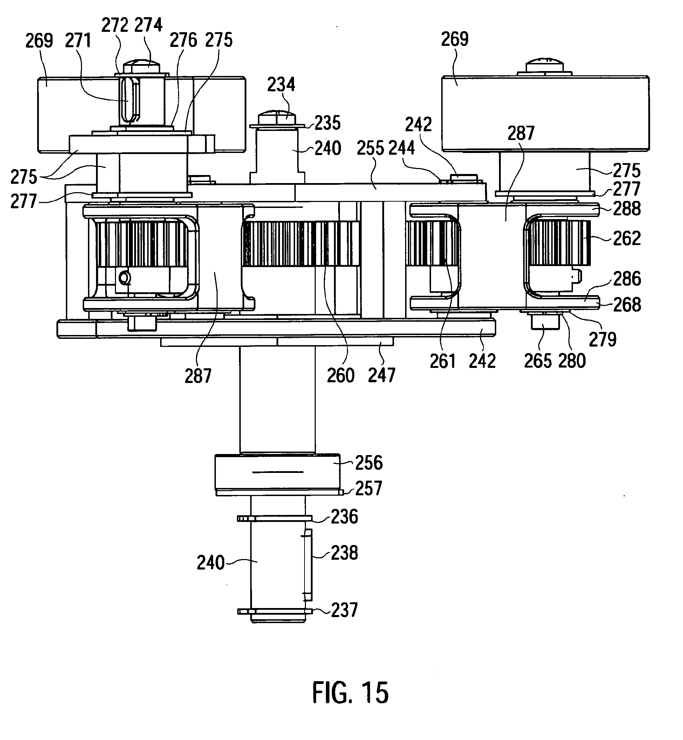

Figure 15 shows a detailed view of the planetary drive which is comprised of the fixed sun gear 260, three closing gears 261 and three roller gears 262. In connection with figure 15 and it should be pointed out that each arm 268 consists of a lower portion 286 and an upper portion 288, which are already comprised in the second embodiment as shown in figure 7. In addition, the lower and upper portions 286 and 288 are integrally formed with two bridging portions 287 for giving the arms more stiffness in order to transmit the force and torque from the rollers 269 to the upper pump unit 250.

Figure 16 shows a top view onto the movable cap 253. The rollers and the respective shafts and sliding blocks are partly assembled. Only one roller 269 is shown, which is fixed by the screw 274, the washer 272 and the feather key 271. On the left-hand side a sliding block 275 is shown. The sliding blocks 275 have a central circular hole for being pivotably fixed to the respective roller shaft 265 by two slide bearings 276, 277. The outer form of the sliding blocks 275 is terete so that the sliding blocks may move a predefined distance radially inward and outward, and still cover the roller holes 292 in the movable cap 255, which have also a terete shape. The roller holes 292 are encompassed by a roller hole frame 293.

Reverting to figure 14, the sliding blocks 275, in particular the downwardly extending rim of the sliding blocks 275 and the roller hole frame 293 prevent spilt over liquids from entering through the roller holes 292 into the planetary drive below. Contrast agents may jam or even etch the gears and consequently damage the roller pump.

Further modifications and variations of the present invention will be apparent to those skilled in the art in view of this description. Accordingly, this description is to be construed as illustrative only and is for the purpose of teaching those skilled in the art the general manner of carrying out the present invention. It is to be understood that the forms of the invention shown and described herein are to be taken as the presently preferred embodiments.

### reference list

- 1: injector head
- 2, 3: handle
- 4: design cap
- 5: hinge splint pin
- 6: bottom
- 21: motor
- 22: gearbox
- 23: gearbox support
- 24: holder
- 25: sleeve portion
- 26: ball bearing
- 27: axial ball bearing
- 28: coupler
- 31: abutment
- 33: internal toothing gear
- 34: columns
- 35: roller carrier
- 36: cylindrical portion
- 37: satellite
- 38: fastening portion
- 39: splint pin
- 40: central shaft
- 41: disposable
- 42: pump hose
- 43: ball bearing
- 44: roller shaft
- 45: sun gear
- 51: roller, conical portion
- 52: rubber roll member
- 53: conical recess
- 54: guidance
- 64: holder
- 65: sleeve portion
- 66: snap ring
- 68: compensating coupler
- 71: motor collar
- 72: collar
- 73: sliding hub
- 100: roller pump
- 110: lower pump unit
- 111: drive axle
- 112: coupling
- 113: driver
- 130: sun gear shaft
- 139: big gear
- 140: closing gear support
- 141: sleeve portion
- 142: support portion
- 150: upper pump unit
- 151: grooved ball bearing
- 152: thin section bearing
- 153: movable cap
- 154: abutment
- 155: inner cap
- 160: sun gear
- 161: closing gear
- 162: roller gear
- 163: sleeve portion
- 164: pin
- 165: shaft
- 166: long hole
- 167: lower arm
- 168: upper arm
- 169: roller
- 170: screw
- 234: lens head screw
- 235: washer
- 236, 237: locking rings
- 238: feather key
- 239: big gear
- 240: drive shaft
- 242: support portion
- 243: spacer
- 244: locking ring
- 245, 246: slide bearing
- 247: slide bearing
- 248: arm shaft
- 249: slide bearing
- 250: upper pump unit
- 251, 256: ball bearing
- 253: movable cap
- 254: abutment
- 255: inner cap
- 257, 258, 259: locking ring
- 260: sun gear
- 261: closing gear
- 262: roller gear
- 265: roller shaft
- 268: arm
- 269: roller
- 271: feather key
- 272: washer
- 274: screw
- 275: sliding block
- 276, 277: slide bearings
- 278, 279: slide bearing
- 280: locking ring
- 281: breaker bolt
- 286: lower portion
- 287: bridging portion
- 288: upper portion
- 292: roller hole
- 293: roller hole frame

## Claims

1. A roller pump comprising:
an abutment (31, 52);
at least one roller (51) for squeezing a pump hose (42) between said roller (51) and said abutment (31, 52), the course of said pump hose (42) defining a plane; and
a casing (2, 3, 6) to which said abutment (31, 52) and said roller (51) are mechanically connected;
**characterized by**
a hinge (5) which connects said abutment (31, 52) and said casing (2, 3, 6) pivotably, the axis of said hinge (5) being parallel to said plane.

2. The roller pump of claim 1, further **characterized by**
a roller carrier (35; 142) to which two rollers (51; 169) are pivotably connected; said roller carrier being pivotable around a central axis with respect to said abutment (31, 52) constituting part of the shell of a cylinder or cone; and
a resilient roll member (52) being fixed to said abutment (31, 52), said two rollers (51) for squeezing a pump hose (42) against said resilient roll member (52).

3. A roller pump comprising:
an abutment (31; 154) constituting part of the shell of a cylinder or cone, said shell or cylinder defining a central axis; and
a roller carrier (35; 142) to which two rollers (51; 169) are pivotably connected; said roller carrier being pivotable around said central axis with respect to said abutment (31; 154);
**characterized by**
a resilient roll member (52) being fixed to said abutment (31, 52), said two rollers (51; 169) for squeezing a pump hose (42) against said resilient roll member (52).

4. The roller pump of one of claims 2 to 3, further **characterized in that**
two roller gears (37; 162), each being torque-proof connected to one of said two rollers (51; 169), the two roller gears (37; 162) engaging with another gear (33; 161, 160) for ensuring zero relative velocity of the portion of said rollers (51; 169) squeezing said pump hose (42) with respect to the squeezed portions of said pump hose (42).

5. A roller pump comprising:
an abutment (31, 52; 154) constituting part of the shell of a cylinder or cone, said shell or cylinder defining a central axis;
a roller carrier (35; 142) to which two rollers (51; 169) are pivotably connected; said roller carrier being pivotable around said central axis with respect to said abutment, said two rollers (51; 169) for squeezing a pump hose (42) against said abutment (31; 154);
**characterized by**
two roller gears (37; 162), each being torque-proof connected to one of said two rollers (51; 169), the two roller gears (37; 162) engaging with another gear (33; 161, 160) for ensuring zero relative velocity of the portion of said rollers (51; 169) squeezing said pump hose (42) with respect to the squeezed portions of said pump hose (42).

6. The roller pump of claims 4 or 5, **characterized in that** said other gear is an internal toothing gear (33); said roller gears (37) further engaging with a sun gear (45) driving said roller gears (37).

7. The roller pump of claims 4 or 5, **characterized in that** each of the roller gears (162) engages with a closing gear (161) and each closing gear (161) further engages with a sun gear (160) being fixed with respect to a housing (110, 150) comprising said abutment (154), each closing gear (161) being pivot mounted on said roller carrier (142).

8. The roller pump of claim 7, **characterized in that** two roller shafts (165) torque-proof connect each of said two rollers (169) with the respective one of said to roller gears (162), each of said roller shafts (165) being pivot mounted within an arm (167, 168), each of said arms (167, 168) being pivot mounted at the axis of the respective closing gear (161).

9. The roller pump of claim 8, further comprising a cap (153) being pivot mounted to said housing (110, 150), said cap (153) being pivotable around said central axis, said roller shafts (165) extending through a long hole (166) in said cap (153), each long hole (166) extending in radial direction with respect to said central axis and limiting the radial movement of the roller shaft (165) extending through the respective long hole (166).

10. The roller pump of one of claims 2 to 9, further comprising:
a motor (21);
a drive train (71, 72, 73) for mechanically connecting said motor (21) and said rollers (51; 169); said drive train (71, 72, 73) comprising a sliding hub (73) for limiting the transmitted torque.

11. A roller pump comprising:
an abutment (31, 52; 154) constituting part of the shell of a cylinder or cone, said shell or cylinder defining a central axis;
a roller carrier (35; 142) to which two rollers (51; 169) are pivotably connected; said roller carrier being pivotable around said central axis with respect to said abutment, said two rollers (51; 169) for squeezing a pump hose (42) against said abutment (31; 154); and
a motor (21);
a drive train (71, 72, 73) for mechanically connecting said motor (21) and said rollers (51; 169);
**characterized in that**
said drive train (71, 72, 73) comprising a sliding hub (73) for limiting the transmitted torque.
